# EUROPÄISCHE PATENTSCHRIFT

(11) **EP 0 804 926 B1**
(45) Veröffentlichungstag und Bekanntmachung des Hinweises auf die Patenterteilung: **27.10.1999**
(21) Anmeldenummer: 97250127.4
(22) Anmeldetag: 19.04.1997
(51) Int. Cl.: A61K 31/565

(54) **Kombinationspräparat zur Behandlung hypogonadaler Männer sowie Männern mit Hypophysenerkrankungen**
Combined preparation for treating male hypogonadism and men with hypophysal disorders
Préparation combinée pour le traitement de l'hypgonadisme et de troubles de la fonction pituitaire de l'homme

(30) Priorität: 02.05.1996 DE 19619045
(43) Veröffentlichungstag der Anmeldung: 05.11.1997
(73) Patentinhaber: Jenapharm GmbH & Co. KG, 07745 Jena (DE)
(72) Erfinder: Oettel, Michael, Prof., 07743 Jena (DE); Golbs, Siegfried, Dr., 04207 Leipzig (DE); Dittel, Michael, Prof., 99510 Apolda (DE); Timpe, Carsten, Dr., 99510 Apolda (DE); Gräser, Thomas, Dr., 99097 Erfurt (DE); Hübler, Doris, Dr., 07407 Schmieden (DE)
(74) Vertreter: Wablat, Wolfgang, Dr.Dr.

(56) Entgegenhaltungen:
- WO-A-94/16709
- DE-A- 2 508 615
- US-A- 4 210 644
- Martindale, JEF Reynolds (ed), 1996, 31. Ausgabe, Seite 1273, Abschnitt "hypogonadism"

## Beschreibung

Die Erfindung betrifft die Verwendung eines pharmazeutischen Kombinationspräparates zur Behandlung hypogonadaler Männer mit und ohne metabolischem Syndrom sowie Männern mit Hypophysenerkrankungen unter oraler, perkutaner, intranasaler, rektaler oder parenteraler Applikation.

In der DE 25 08 615 A1 ist die Veresterung von Testosteron mit Fettsäuren bestimmter Kettenlänge beschrieben, wobei die orale Wirksamkeit des an sich oral unwirksamen Testosterons verbessert werden soll. Es wird die Beseitigung des Testosteronmangels im Alter (bei Impotenz, Klimakterium virile) durch Gabe von Testosteron und Estrogen beschrieben. Die Behandlung von Hypophysenerkrankungen ist nicht offenbart.

Die U.S.-PS 4,210,644 offenbart eine Androgen-Estrogen-Kombination zur Fertilitätskontrolle und zur Libidosteigerung. Die Verwendung dieser Kombination bei hypogonodalen Männern sowie Männern mit Hypophysenerkrankungen ist nicht beschrieben.

Verschiedene endokrine Funktionen verändern sich im Verlauf des Alterungsprozesses. So nehmen z. B. die Blutplasma-Konzentrationen an Insulin-like growth factor (IGF-1) mit zunehmenden Alter bei gesunden Personen ab (Rudman D(1985): Growth hormone, body composition, and aging. J Am Geriatr Soc 33: 800-807; Florini JR, Prinz PN, Vitiello MV, Hintz RL (1985): Somatomedin-C levels in healthy young and old men: Ralationship to peak and 34-hour integrated levels of growth hormone. J. Gerontol 40: 2-7). Der normale Alterungsprozess ist beim Mann mit einer Abnahme der Hodenfunktion, insbesondere mit einer Abnahme der Serum-Testosteron-Spiegel gekoppelt. Gleichzeitig kommt es zum Anstieg der Luteinisierungshormon (LH)- und Follikel-stimulierenden Hormon- (FSH)- Konzentrationen im Blutplasma (Tien-Chun Chang, Chin-Chia Tung, Yung-Lien Hsiao (1994): Hormonal changes in elderly men with non-insulin-dependent diabetes mellitus and the hormonal relationships to abdominal adiposity. Gerontology 40: 260-267).

Parallel zu diesen hormonellen Veränderungen steigt die Prävalenz an bestimmten Erkrankungen mit und ohne gestörter oraler Glukosetoleranz an. Beispiele hierfür sind die verschiedenen Formen des Diabetes mellitus, Bluthochdruck, Hypercholesterolämie und andere Störungen des Lipid- bzw. Lipoprotein-Stoffwechsels, Myokardinfarkt und Morbus Alzheimer (Vermeulen A (1991): J. Clin Endocrinol Metab 73: 222). Sehr häufig wird bei älteren übergewichtigen Männern das sogenannte Metabolische Syndrom diagnostiziert, das mit Obesitas, Insulin- bzw. Insulinrezeptor-Resistenz, Testosterondefizit und einem überdurchschnittlich hohem Risiko für Herz-Kreislauferkrankungen einhergeht. Die Mortalität infolge cerebraler oder koronarer Ischämie ist enorm erhöht (McGovern PG et al. (1993): The role of stroke attack rate and case fatality in the decline of stroke mortality. The Minnesota Heart Survey. Ann Epidemiol. 3: 483-487; Hames CG et al. (1993): Black-white comparisons of 20-year coronary heart disease mortality in the Evans County Heart Study Cardiology 82: 122-136).

Im allgemeinen werden Patienten mit Serum-Testosteron-Konzentrationen mit regelmäßig weniger als 3,5 ng/ml nach wiederholter Bestimmung als hypogonadal bezeichnet und mit geeigneten Androgenen im Sinne einer Hormonsubstitution behandelt.

Die Testosteron-Substitution kann oral, intramuskulär oder transdermal (z. B. transskrotal) erfolgen. Dabei ist jedoch zu beachten, daß Testosteron selbst entweder durch Bindung an einen spezifischen Androgenrezeptor in speziellen Targetgeweben wirkt, zu Estradiol aromatisiert wird oder zu Dihydrotestosteron reduziert werden kann und daher die Wirkung des Testosterons im wesentlichen vom Ausmaß der Aromatisierung bzw. der Reduzierung abhängig ist. In bestimmten Organen wie Muskulatur, Skelett und Hoden wirkt Testosteron direkt am Androgenrezeptor. In verschiedenen peripheren Geweben, wie externe Genitalien, akzessorische Geschlechtsdrüsen (z. B. Prostata) und der Haut, muß Testosteron erst zu 5α-Dihydrotestosteron reduziert werden, um seinen androgenen Effekt entfalten zu können. 5α-Dihydrotestosteron ist ca. zweimal aktiver als Testosteron. In anderen Geweben, wie Fettgewebe und in bestimmten Hirnarealen bzw. -zellen, wird Testosteron zum weiblichen Geschlechtshormon Estradiol aromatisiert und wirkt dann über den Estrogenrezeptor.

Bei gesunden jungen Männern werden im Blut 3,0 bis 10,0 ng/ml Gesamt-Testosteron, 2 % des Gesamt-Testosteron (0,06 bis 0,2 ng/ml) als ungebundenes sogenanntes "freies" Testosteron, 0,25 bis 0,75 ng/ml 5α-Dihydrotestosteron und <5Opg/ml 17β-Estradiol gemessen (Ikuko Kato et al. (1992) : Determinants of sex hormone levels in men as useful idices in hormone-related disorders. J. Clin Epidemiol. 45:1417-1421; Andersson SO et al. (1993): Serum pituitary and sex steroid hormone levels in the ethiology of prostatic cancer-a population-based case-control study. Br. J. Cancer 68: 97-102; Mikuma N et al. (1994) : Role of the hypothalamic opioidergic system in the control of gonadotropin secretion in elderly men. Andrologia 26:39-45).

Dieses gut ausbalancierte Verhältnis zwischen der "Muttersubstanz" Testosteron und seiner beiden Hauptstoffwechselmetaboliten 17β-Estradiol und 5α-Dihydrotestosteron ist für die Aufrechterhaltung der Homöostase des Mannes und damit für die Funktionsfähigkeit wichtiger Regelkreise für Protein-, Kohlenhydrat- und Lipoproteinstoffwechsel, für das hämatopoetische System incl. Fibrinolyse, für die Aufrechterhaltung der Knochen- und Muskelmasse, für die Haut- und Leberfunktion und für bestimmte Verhaltensweisen essentiell. Damit wird auch deutlich, daß eine Störung dieser Balance bestimmte Krankheitszustände nach sich ziehen kann.

Testosteron und Estradiol können in sehr komplexer Art und Weise interagieren. In bestimmten Geweben vermehrt Estradiol die Konzentration an Androgenrezeptoren und potenziert damit den Androgeneffekt (synergistische Wirkung), während z. B. in den Mammae von Mäusen Testosteron als ein Estradiol-Antagonist wirkt. Darüber hinaus können Androgene in Brustkrebs-Zellinien (MCF6-Zellen) am Estrogenrezeptor angreifen und damit die estrogenabhängige Progesteronrezeptor-Synthese hemmen (antagonistischer Effekt).

Aber auch die Art und Weise, wie ein Androgenmolekül synthetisch verändert wird, kann den Grad der Umwandlung in Estrogene beeinflussen und damit das pharmakodynamische Profil des jeweiligen Androgens grundlegend bestimmen. Die oral wirksamen 17α-alkylierende Androgene können nicht oder nur schwerlich zu Estrogenen aromatisiert werden. Sie senken das HDL-Cholesterol, das HDL₂-Cholesterol und die Apolipoproteine AI und AII. Sie bewirken einen Anstieg des Gesamt- und LDL-Cholesterol, des Apolipoprotein B, der hepatischen Triglycerid-Lipase und der Lipoprotein-Lipase. Im Gegensatz zu den 17α-alkylierten Androgenen haben Testosteronester, wie z. B. Testosteronenanthat, nur einen geringen Effekt auf das Gesamt- und LDL-Cholesterol, obwohl sie eine geringere Suppression von HDL-Cholesterol bewirken können. Diese Differenz zwischen 17α-alkylierten und nicht alkylierten Androgenen (z. B. Testosteronester) ist auf die Fähigkeit des Testosterons bzw. seiner Ester zur Aromatisation zu Estrogenen zurückzuführen. Estrogene besitzen ja bekanntermaßen einen gegensätzlichen Effekt wie die Androgene auf die LDL- und HDL-Cholesterolkonzentrationen.

Bei älteren Männern verändert sich jedoch das Stoffwechselmuster des Testosterons sehr erheblich, d. h. die oben geschilderte Balance zwischen Testosteron, 5α-Dihydrotestosteron und 17β-Estradiol wird nachhaltig gestört. Dabei verändern sich die quantitativen Aspekte des Testosteronmetabolismus, es werden mehr Estrogene und weniger 5α-reduzierte Metaboliten gebildet.

Besonders bei adipösen Männern ist die Aromatisierung zu Estradiol erhöht (De Lignière B (1993) Transdermal dihydrotestosterone treatment of andropause, Ann Med 25: 235-241). Wird Testosteron bzw. dessen Fettsäureester an hypogonadale Männer appliziert, so wird im Vergleich zu gesunden Männern ein viel höherer Anteil zu Estradiol aromatisiert. Es kommt nach perkutaner Applikation zu einem um 70 % höheren Anstieg der Estradiol-Plasmakonzentrationen (Kuhn JM Laudat MH, Lignières de B, Bricaire H. Lutaon JP (1986) Traitement androgénique percutané des hypogonadismes masculins. Efficacité comparée de la testosterone et de la dihydrotestostérone: étude de 40 observations. Contracept Fert Sex 14: 1031-1036). Nach intramuskulärer Injektion steigen die Estradiol-Plasmakonzentrationen sogar auf das Doppelte an (Tenover JS (1992) Effects of testosterone supplementation in the aging male. J. Clin Endocrinol Metab 75: 1092-1098). Dies führt bei vielen Patienten zu Estradiol-Konzentrationen, die denen bei Frauen in der Follikelphase gleichen. Und so ist es kein Wunder, daß bei hypogonadalen, adipösen Männern die Testosteronapplikation zu einer Reihe von unerwünschten Effekten wie z. B. Auslösung oder Verstärkung einer Gynäkomastie und relativer peripherer Androgenmangel (Verminderung der Libido, Verkleinerung der Geschlechtsorgane, aber auch Prostatahyperplasie) führen kann.

Die exogene Zufuhr von Testosteron, z. B. Testosteronenanthat, bei Patienten mit noch vorhandener gonadaler Testosteronbiosynthese, inhibiert nach kurzer Zeit reversibel die körpereigene Testosteronproduktion über einen Zeitraum von etwa 2 Wochen (Hümpel M. und Oettel M. (1994): Wertende Zusammenfassung Pharmakokinetik und Biotranformation von Testosteronenanthat beim Menschen). Dies wird nach Applikation von Estradiol bzw. Dihydrotestosteron (DHT) oder Mesterolon nicht beobachtet (Oettel M. (1993): in Hagers Handbuch der Pharmazeutischen Praxis Band 7 und 8).

Hinzu kommt, daß im Alter auch der reduktive Stoffwechsel zu dem androgen hochaktiven Metaboliten 5α-Dihydrotestosteron gestört sein kann. Statt des 5α-Stoffwechselweges wird das 5β-Metabolisierungsmuster verstärkt, d. h. es entstehen viel schwächer androgenwirksame Metabolite. Dies hat dazu geführt, daß Androgene in enorm hohen Dosen (z. B. oral 400 bis 600 mg Testosteronundecanoat/Tag oder 200 bis 250 mg Testosteronenanthat i. m. aller 2 bis 3 Wochen) appliziert werden müssen, um die gewünschten androgenen Effekte erzielen zu können. Damit erhöhen sich zwangsläufig auch die Estradiolkonzentrationen mit den o. g. unerwünschten Nebenwirkungen.

Nach Yarnell JWG et al. 1993 (Arterioscl. Thromb. 13: 517-520) sind bei Männern mit schwerer ischämischer Herzkrankheit die Estradiol-Blutplasma-Konzentrationen leicht erhöht. In dieser sogenannten Caerphilly Prospective Study bewegten sich bei insgesamt 2161 gesunden Kontrollpersonen im Alter von 45 bis 59 Jahren die Plasma-Estradiol-Konzentrationen bei 69+/-17 pg/ml (250+/-61pmol/l) und bei 134 Männern im gleichen Alter mit schwerer ischämischer Herzkrankheit bei 71+/-19pg/ml (257+/-69pmol/l).

Allerdings kann auf die Vorzüge des Estradiols in niedriger Konzentration nicht verzichtet werden (wie dies bei Gabe des nicht-aromatisierbaren Dihydrotestosterons oder seiner Derivate der Fall ist). Diese Vorteile beziehen sich u. a. auf die Verbesserung cognitiver Leistungen, auf die Erhöhung des Sexualhormon-bindenden Globulins (SHBG), auf die Hemmung der Oxidation des LDL-Cholesterols als wichtigen Schritt innerhalb der Atherogenese, auf die Weitstellung von Arterien und die damit verbundene bessere Duchblutung der Gewebe und auf die Hemmung der erhöhten Gonadotropin- und Inhibin-Konzentrationen (Harman SM. Blackman MR. (1994) Male menopause, myth or menace? Endocrinologist 4:212-217). Weiterhin scheint Estradiol altersabhängige Umbauvorgänge bei Proteohormonen zu verhindern (Wide L. Maessen T. (1994) 17β-Estradiol counteracts the formation of the more acidic isoforms of follicle-stimulating hormone and luteinizing hormone after menopause. Clin Endocronol 40: 783-789).

Auch die Wirksamkeit des unveränderten Testosterons nimmt im Alter deutlich ab, da das wichtigste Transportprotein, das Sexualhormon-bindende Globulin (SHBG) durch strukturelle Umbauvorgänge das Testosteron viel fester bindet. Die Folge ist ein geringerer Anteil des freien, d. h. des biologisch aktiven Testosterons. Im Gegensatz dazu zeigen 5α-Dihydrotestosteron und Estradiol keinen deutlichen Altersgang (Vermeulen A. (1972) Testosterone secretion and metabolism in male senescence. J Clin Endocrinol 34: 730-735).

Aufgabe der vorliegenden Erfindung ist es, durch die Verwendung geeigneter pharmazeutischer Zubereitungsformen die Auslenkungen des Testosteronmetabolismus, bei Hypophysenerkrankungen, sowie bei Hypogonadismus mit und ohne metabolischem Syndrom zu vermeiden und damit die o. g. Balance zwischen Androgenen und Estradiol bei gesunden jungen Männern auch im Alter zu erhalten.

Diese Aufgabe wird erfindungsgemäß durch die Verwendung eines Kombinationspräparats zur Behandlung der Auslenkungen des Testosteronmetabolismus hypogonadaler Männer sowie Männern mit Hypophysenerkrankungen gelöst, welches ein biogenes oder synthetisches Androgen und ein biogenes oder synthetisches Estrogen enthält. Die Patienten erhalten dabei exogen die Endprodukte des Testosteronmetabolismus, d.h. ein 5α-reduziertes Testosteron/Estrogenpräparat in einem ähnlichen oder gleichen Verhältnis, wie dieses natürlicherweise bei jüngeren und gesunden Männern auftritt.

Die erfindungsgemäße Verwendung ist dadurch gekennzeichnet, daß das biogene Androgen mindestens einen Bestandteil aus der Gruppe Testosteron, Androsteron, Androstanolon und anderen biogenen Androgenen oder mindestens eine Verbindung, die einen der vorgenannten Androgenbestandteile nach Einnahme rasch abspaltet, aufweist.

Bevorzugt ist ein erfindungsgemäß verwendetes Kombinationspräparat, wobei das biogene Androgen 5α-Dihydrotestosteron oder eine Verbindung, die 5α-Dihydrotestosteron nach Einnahme rasch abspaltet, ist.

Das erfindungsgemäß verwendete Kombinationspräparat ist ferner dadurch gekennzeichnet, daß das synthetische Androgen mindestens einen Bestandteil aus der Gruppe Mesterolon, Fluoxymesteron, 17-Methyltestosteron und anderen synthetischen Androgenen oder mindestens eine Verbindung, die einen der vorgenannten Androgenbestandteile nach Einnahme schnell abspaltet, aufweist.

Das erfindungsgemäß verwendete Kombinationspräparat ist auch dadurch gekennzeichnet, daß das biogene Estrogen mindestens einen Bestandteil aus der Gruppe 17β-Estradiol, Estron, Estriol, Estran und anderen biogenen Estrogenen oder mindestens eine Verbindung, die einen der vorgenannten Estrogenbestandteile nach Einnahme schnell abspaltet, aufweist.

Das erfindungsgemäß verwendete Kombinationspräparat ist ferner dadurch gekennzeichnet, daß das synthetische Estrogen mindestens einen Bestandteil aus der Gruppe Ethinylestradiol, Mestranol und anderen synthetischen Estrogenen oder mindestens eine Verbindung, die eine der vorgenannten Estrogenbestandteile nach Einnahme schnell abspaltet, aufweist.

Geeignete Verbindungen, welche die biogenen oder synthetischen Androgene oder die biogenen oder synthetischen Estrogene nach Einnahme schnell abspalten sind Ester, Ether, Amide und Salze der Androgene oder Estrogene mit pharmazeutisch verträglichen Säuren, Alkoholen oder Aminen. Geeignete Säuren sind Mineralsäuren, wie beispielsweise Schwefelsäure, Salzsäure, Phosphorsäure, oder organische Carbonsäuren mit bis zu 25 C-Atomen, wie beispielsweise Essigsäure, Ameisensäure, Propionsäure, Valeriansäure, Benzoesäure oder Fettsäuren, wie Linolsäure, Linolensäure, Ölsäure, Myristinsäure, Undecanonsäure. Geignete Alkohole sind beispielsweise aliphatische Alkohole mit bis zu 15 C-Atomen, wie Ethanol, Methanol, Propanol, oder aromatische Alkohole, wie Phenole. Geeignete Amine sind aliphatische Amine mit bis zu 8 C-Atomen. Vorteilhafterweise können auch α- oder β-Aminocarbonsäuren zur Veresterung, Veretherung oder Amidbildung eingesetzt werden. Die Herstellung erfolgt in an sich bekannter Weise.

Die Aufgabe wird erfindungsgemäß durch die Verwendung von Kombinationen von Estrogenen und und Androgenen bzw. deren Derivaten in unterschiedlichen Zubereitungs- bzw. Applikationsformen gelöst. Dadurch werden unphysiologische Veränderungen steroidwandelnder Enzyme (Aromatasen, Reduktasen) vermieden und die Therapie wesentlich verbessert.

Das erfindungsgemäß verwendete Kombinationspräparat zur Behandlung hypogonadaler Männer mit und ohne metabolischem Syndrom sowie Männern mit Hypophysenerkrankungen besteht aus fixen Kombinationen von 5α-Dihydrotestosteron, seinen verschiedenen Estern und synthetischen Abkömmlingen mit natürlichen oder synthetischen Estrogenen und deren verschiedenen Estern und Derivaten.

Erfindungsgemäß wird bevorzugt als Derivat des Dihydrotestosterons das Mesterolon eingesetzt.

Das natürliche Estrogen weist erfindungsgemäß mindestens einen Bestandteil aus der Gruppe 17β-Estradiol, Estron, Estriol oder konjugierten Estrogenen auf.

Das synthetische Estrogen weist erfindungsgemäß mindestens einen Bestandteil aus der Gruppe Ethinylestradiol oder Mestranol auf.

Die pharmazeutischen Zubereitungsformen können die Kombination als einheitliche Form darstellen oder auch zwei getrennte Formen beinhalten. Dabei können es sich um Peroralia, wie z. B. Tabletten, Kapseln und Dragees, um perkutane Zubereitungsformen wie z. B. Transdermale Therapeutische Systeme (TTS) oder Gele, Sprays oder Salben, um intranasale Zubereitungsformen wie Nasensprays oder Nasentropfen, rektale Zubereitungsformen wie Suppositorien und um Parenteralia wie z. B. Implantate bzw. Presslinge und Ampullen handeln.

Die Zubereitungsformen werden in an sich üblicher Weise unter Verwendung üblicher Hilfs- und Trägerstoffe hergestellt, wie sie beispielsweise im "Remington's Pharmaceutical Sciences Handbook, Hack Pub. Co., N. Y., USA" beschrieben sind.

Eine Variante der Applikation der erfindungsgemäßen Wirkstoffkombination stellt die Applikation in Form von Zäpfchen oder Kapseln dar. Dazu werden Zäpfchen und Kapseln nach den üblichen Methoden unter Verwendung der üblichen Hilfsstoffe hergestellt.

Das erfindungsgemäß verwendete Kombinationspräparat kann ferner in Form von transdermalen therapeutischen Systemen (TTS) appliziert werden. Dazu werden die erfindungsgemäß verwendeten Wirkstoffkombinationen in an sich bekannter Weise in ein TTS eingebracht. Das TTS kann beispielsweise auf Iontophorese oder Diffusion oder gegebenenfalls auf Kombinationen dieser Effekte beruhen. Das TTS wird an geeigneter Stelle am Körper angebracht. Die Wirkstoffe werden dann transcutan appliziert, wobei die Applikationsrate durch die Größe der Fläche des TTS und die gegebenenfalls angelegte Spannung gesteuert wird.

Für die bevorzugte orale Applikation werden die erfindungsgemäß verwendeten Kombinationspräparate zweckmäßig in Form einer pharmazeutischen Packung zusammengefaßt, welche die tägliche Dosierung darstellende Formulierung unterbringt.

Nachfolgend sind einige Beispiele für pharmazeutische Zubereitungen des erfindungsgemäßen Kombinationspräparates aufgezeigt:
- Mesterolon-Tablette (25 mg) plus 0,80 mg Estradiolvalerat
- DHT-Gel (125 mg/dosi in 2,0 ml) plus 1,0 mg mikronisiertem Estradiol als Gel
- Mesterolon-Tablette (25 mg) plus Estradiol-Pflaster (0,025 mg Estradiol/Tag)
- DHT-enanthat 12,5 mg i.m. und 0,025 mg EV i.m. (als eine Ampulle)
- DHT (125 mg) plus Estradiol (1 mg) - als Spray
- Mesterolon Tablette (25 mg) plus 0,80 mg Estradiolvalerat als Kapsel
- DHT (125 mg) plus 5 mg Estriol als Gel oder Pflaster
- Mesterolon Tablette (25 mg) plus 0,50 mg Estradiolvalerat als Suppositorium
- DHT (125 mg) plus 5 mg Estriol als Nasenspray bzw. Tropfen
- Mesterolon-Tabletten (25 mg) plus 0,60 mg konjugiertes Estrogen

Weitere galenische Zubereitungsformen, die beliebig kombiniert werden können, sind beispielsweise:
- Bioabbaubare Microspheren mit einem Gewicht von 300 mg und einem DHT-Gehalt von 150 mg, die täglich 6 mg DHT freisetzen, kombiniert mit:
   - 0,80 mg Estradiolvalerat (Dragee, Tablette)
   - Estradiol-Gel (1 mg mikronisiertes Estradiol)
   - Estriol Gel (5 mg Estriol)
   - Estradiol-Pflaster (0,25 Estradiol/Tag)
   - 0,60 mg konjugiertes Estrogen (Dragee, Tablette)
- DHT-Heptanoat 100 mg bis 200 mg i. m. kombiniert mit:
   - 0,80 mg Estradiolvalerat (Dragee, Tablette)
   - Estradiol-Gel (1 mg mikronisiertes Estradiol)
   - Estriol-Gel (5 mg)
   - Estradiol-Pflaster (0,25 mg Estradiol/Tag)
   - 0,60 mg konjugiertes Estrogen (Dragee, Tablette)

### Beispiel 1

### Pharmakokinetisches Verhalten von Mesterolon in Kombination mit Estradiolvalerat (EV)

In einer randomisierten Studie an 24 gesunden jungen männlichen Probanden wurde das kinetische Verhalten von Mesterolon (25 mg Tablette) in Kombination mit EV (1 mg Dragee) nach einmaliger oraler Gabe untersucht.

Nach einer kontrollierten Fastenperiode von 12 Stunden erhielt jeder Proband Mesterolon und EV verabreicht. Es wurden nach der Applikation über einen Zeitraum von 60 Stunden von jedem Probanden 18 Blutproben entnommen.

Für das Mesterolon wurden folgende Ergebnisse zum pharmakokinetischen Verhalten ermittelt:

| | |
|---|---|
| AUC | 176,8 ng/ml*h |
| Cₘₐₓ | 6,9 ng/ml |
| tₘₐₓ | 8,9 h |
| t_{1/2} | 13,2 h |

Nach 60 Stunden war mit 0,48 ng/ml der 0-Wert annähernd erreicht.

Folgende pharmakokinetischen Ergebnisse konnten nach Analyse des Estradiols ermittelt werden:

| | freies Estradiol | freies Estron | konjugiertes Estron |
|---|---|---|---|
| AUC (pg/ml*h) | 970,8 | 3492,3 | 128701,8 |
| Cₘₐₓ (pg/ml) | 38,7 | 164,0 | 15171,0 |
| tₘₐₓ (h) | 7,8 | 7,0 | 2,4 |
| t_{1/2} (h) | 15,9 | 15,7 | 12,5 |

Nach 24 Stunden lag der Anteil von freiem Estradiol noch bei 28 pg/ml.

### Beispiel 2

### Pharmakokinetisches Verhalten von Dihydrotestosteron (DHT) in Kombination mit Estriol-Gel

In einer randomisierten Studie an 18 gesunden jungen männlichen Probanden wurde das pharmakokinetische Verhalten von DHT und Estriol nach transdermaler Applikation untersucht.

Das DHT-Pflaster enthält 150 mg Wirkstoff und wurde über 24 Stunden auf der Haut der Probanden belassen. Diese galenische Form setzt täglich 6 mg DHT frei. Die Ausgangswerte für DHT lagen bei den Probanden zwischen 0,43 und 1,24 ng/ml.

Das Estriol-Gel enthält 5 mg Wirkstoff in 0,5 g Gel. Diese Gel-Formulierung wurde auf 150 cm² Haut (Unterarm) einmalig aufgetragen. Die Estriol Ausgangswerte lagen bei allen Probanden unter der Nachweisgrenze von <3 pg/ml.

Über einen Zeitraum von 48 Stunden Post applikationem wurden je Probanden 12 Blutproben für Analysezwecke entnommen. Für DHT konnte ein Cₘₐₓ-Wert von 4,51+/-1,23 ng/ml ermittelt werden. Die Cₘₐₓ-Konzentrationen für Estriol lagen bei 8,82+/-2,54 pg/ml.

Vergleicht man die Ergebnisse der pharmakokinetischen Untersuchungen aus Beispiel 1 und 2 mit den Ergebnissen einer Studie mit gesunden jungen Männern, die Testosteronenantat (250 mg i. m.), kombiniert mit Estradiol-Pflaster (25 mg) appliziert bekamen, so wird deutlich, daß hohe Cmax-Werte von 19,4 +/-7,9 ng/ml nach 2,4 Tagen für Testosteron und für Estradiol von 148,1 +/-26,3 pg/ml nach 2,7 Tagen erreicht werden. Die in dieser Studie ermittelten hohen Estradiolkonzentrationen reflektieren die späte Follikelphase bei der Frau.

### Beispiel 3

### Klinische Studie mit 23 hypogonadalen Männern

Die Studie wurde zweiarmig an 23 hypogonadalen Männern, deren Testosteronspiegel unter 3ng/ml Testosteron lag, durchgeführt.
Arm 1 (n=12) 250 mg Testosteronenanthat i. m. alle 3 Wochen über 12 Monate
Arm 2 (n=11) 25 mg Mesterolon/Tag p. o. plus 0,8 mg Estradiolvalerat/Tag p. o. über 12 Monate

Im Ergebnis der Studie sind folgende Resultate ermittelt worden:
- Arm 1:: Nach i. m. Applikation von 250 mg Testosteronenanthat wurden 2 bis 3 Tage nach dem jeweiligen Applikationszeitpunkt Testosteronmaximalwerte von 22,7 +/-14,8 ng/ml im Serum nachgewiesen. Nach 21 Tagen lagen die Werte noch bei 6,7 +/-2,1 ng/ml. Bei den nachfolgenden Applikationen wurden ähnliche Ergebnisse gefunden. Eine Kumulation war nicht vorhanden. Vergleicht man die Maximalwerte mit den bei gesunden Männern nachgewiesenen Testosteronblutspiegeln, so ist eine deutliche Erhöhung erkennbar, so daß unerwünschte Ereignisse nicht zu erwarten sind.
Durch Inhibierung der noch in geringem Umfang vorhandenen körpereigenen Testosteronsynthese war eine Abnahme der erhöhten LH-Konzentrationen im Serum bei allen Studienteilnehmern zu beobachten.
Bei allen Patienten konnte bereits nach 6 Monaten Behandlungsdauer eine deutliche Knochendichtezunahme nachgewiesen werden. Die erhöhten Testosteronspiegel führten bei allen Männern zu einer Zunahme der Körperbehaarung.
Eine Verbesserung der morgendlichen Peniserektion war von 9 der 12 Patienten beobachtet worden. Im Verlauf der Studie war eine Penisgrößenzunahme bei 11 Männern nachweisbar bei gleichzeitiger Reduzierung des Hodenvolumens.
Symptome des Klimakterium virile, wie Hot Flushing, Müdigkeit, Konzentrationsschwäche mit Nachlassen der Leistungsfähigkeit, Verstimmung, Libido- und Potenzstörungen, traten weniger häufig in Erscheinung. 9 Patienten waren beschwerdefrei.
Die Werte des Prostata-spezifischen Antigens (PSA) waren bei allen Patienten leicht erhöht. Sie lagen zwischen 3,0 und 3,8 ng/ml. In diesem Zusammenhang war aber keine Zunahme der Prostatae nachweisbar.
Ein Hautkollagenschwund konnte nicht beobachtet werden. Es war eine Hauverdickung mit vermehrter Wassereinlagerung und Verbesserung des Turgor und der Elastizität bei allen Studienteilnehmern nachweisbar.
Bei 7 der 12 Patienten konnte nach 4 Behandlungsmonaten eine erhöhte Bereitschaft zur Ausbildung von Akne beobachtet werden. Gleichzeitig war bei allen Männern eine Veränderung der Stimmlage zum Baß hin zu beobachten.
Bei 5 von 12 Patienten bildete sich eine Gynäkomastie aus.
- Arm 2:: Nach täglicher Verabreichung von Mesterolon (25 mg) und Estradiolvalerat (0,80 mg) über 12 Monate an 11 hypogonadale Männer konnten Maximalkonzentrationen von DHT (5,2+/-2,0 ng/ml) 9 Stunden post applikationem ermittelt werden. Nach 24 Stunden waren noch 1,82 +/-0,85 ng/ml nachweisbar. Bei täglicher Applikation sank der DHT-Spiegel im Verlauf der Studie bei keinem Patienten unter 1,2 ng/ml. Kumulative Effekte sind nicht aufgetreten. Diese ermittelten Konzentrationen können als therapeutisch wirksame Spiegel bei der Behandlung hypogonadaler Männer angesehen werden.

Maximalkonzentrationen von Estradiol (32,4 +/-7,8 pg/ml) konnten 7,9 Stunden nach der ersten Applikation nachgewiesen werden. Dieser Effekt wiederholte sich regelmäßig nach jeder Applikation, wobei keinerlei Kumulation zu beobachten war. Die Maximalkonzentrationen für Estron lagen 6,7 Stunden nach der 1. Applikation bei 154,2 +/-29,8 pg/ml. Auch beim Estron gab es keine Kumulation.

Mit den vorgefundenen Estrogenkonzentrationen sind die estrogenbedingten Störungen im Klimakterium virile therapierbar. Schlafstörungen traten nach 3 Behandlungsmonaten bei keinem Patienten mehr auf. Da durch Estrogene auch die Sebumsynthese gehemmt wird, war bei keinem Studienteilnehmer eine Akneausbildung beobachtet worden.

Da keine LH-Synthesehemmung vorlag, kam es bei 9 Patienten zu einem leichten Anstieg der Testosteronkonzentrationen auf maximal 11,3 ng/ml. Zwischen Testosteronblutspiegel und Knochenmineralisation bestand eine positive Korrelation, so daß bereits nach 6 Therapiemonaten bei allen Patienten eine deutliche Knochendichtezunahme zu verzeichnen war.

Bei allen 11 Studienteilnehmern waren die Symptome des Klimakteriums virile nach spätestens 4 Monaten abgeklungen bzw. nicht mehr vorhanden.

Eine Größenzunahme des Penis sowie eine leichte Erhöhung der Hodenmasse war bei 10 Patienten vorhanden. Eine Zunahme der Körperbehaarung war ebenfalls bei allen Männern zu beobachten.

Eine Prostatahyperplasie war bei keinem Patienten nach 12 Behandlungsmonaten ersichtlich.

Im Gegensatz zur alleinigen Testosteronbehandlung kam es bei der Kombinationstherapie zu keinem PSA-Anstieg, keiner Veränderung der Stimmlage und Gynäkomastie.

## Patentansprüche

1. Verwendung einer Kombination aus einem biogenen oder synthetischen Androgen und einem biogenen oder synthetischen Estrogen zur Herstellung eines Medikaments zur Behandlung von hypogonodalen Männern sowie Männern mit Hypophysenerkrankungen zur Vermeidung der Auslenkungen des Testosteronmetabolismus.

2. Verwendung nach Anspruch 1, dadurch gekennzeichnet, daß das biogene Androgen eine der Substanzen Androsteron, Androstanolon, 5α-Dihydrotestosteron oder ein anderes biogenes Androgen ist oder eine Verbindung darstellt, die einen der vorgenannten Androgenbestandteile nach Einnahme rasch abspaltet.

3. Verwendung nach Anspruch 1, dadurch gekennzeichnet, daß das synthetische Androgen eine der Substanzen Mesterolon, Fluoxymesteron oder ein anderes synthetisches Androgen ist oder eine Verbindung darstellt, die einen der vorgenannten Androgenbestandteile nach Einnahme rasch abspaltet.

4. Verwendung nach Anspruch 1, dadurch gekennzeichnet, daß das biogene Estrogen eine der Substanzen 17β-Estradiol, Estron, Estriol, Estran oder ein anderes biogenes Estrogen ist oder eine Verbindung darstellt, die einen der vorgenannten Estrogenbestandteile nach Einnahme rasch abspaltet.

5. Verwendung nach Anspruch 1, dadurch gekennzeichnet, daß das synthetische Estrogen eine der Substanzen Ethinylestradiol, Mestranol oder ein anderes synthetisches Estrogen ist oder eine Verbindung darstellt, die einen der vorgenannten Estrogenbestandteile nach Einnahme rasch abspaltet.

6. Verwendung nach mindestens einem der Ansprüche 1 bis 5 dadurch gekennzeichnet, daß man das Medikament in Form von Tabletten, Kapseln, Dragees, Transdermalen-Therapie-Systemen, Ampullen, Suppositorien, Gelen, Salben, Nasentropfen, Implantaten, Presslingen oder bioabbaubaren Mikrospheren herstellt.

## Claims

1. Use of a combination of a biogenous or synthetic androgen and a biogenous or synthetic estrogen to produce a pharmaceutical for the treatment of hypogonadal men and men with hypophyseal diseases to prevent the imbalances of testosterone metabolism.

2. The use according to claim 1 wherein the biogenous androgen is at least one substance from the group of androsterone, androstanolon, 5α-dihydrotestosterone or another biogenous androgen, or a compound that splits off one of the above androgen components shortly after application.

3. The use according to claim 1 wherein the synthetic androgen is at least one substance from the group of mesterolone, fluoxymesterone or another synthetic androgen, or a compound that splits off one of the above androgen components shortly after application.

4. The use according to claim 1 wherein the biogenous estrogen is at least one substance from the group of 17β-estradiol, estrone, estriol, estrane or another biogenous estrogen, or a compound that splits off one of the above estrogen components shortly after application.

5. The use according to claim 1 wherein the synthetic estrogen is at least one substance from the group of ethinyl estradiol, mestranol or another synthetic estrogen, or a compound that splits off one of the above estrogen components shortly after application.

6. The use according to at least one of claims 1 to 5 wherein said pharmaceutical is produced in the form of tablets, capsules, lozenges, transdermal therapy systems, ampules, suppositories, gels, ointments, nasal drops, implants, compacts or biodegradable microspheres.

## Revendications

1. Utilisation d'une association d'un androgène, d'origine biologique ou de synthèse, et d'un oestrogène, d'origine biologique ou de synthèse, pour la fabrication d'un médicament pour le traitement de l'hypogonadisme et des troubles de l'hypophyse de l'homme, visant à éviter les dysfonctionnements du métabolisme de la testostérone.

2. Utilisation selon la revendication 1, caractérisée en ce que l'androgène d'origine biologique est choisi parmi l'androstérone l'androstanolone, la 5α-dihydrotestostérone ou un autre androgène d'origine biologique ou est un composé qui se dégrade rapidement après administration, pour libérer un des composants androgènes précédemment cités.

3. Utilisation selon la revendication 1, caractérisée en ce que l'androgène de synthèse est choisi parmi la mestérolone, la fluoxymestérone ou un autre androgène de synthèse ou est un composé qui se dégrade rapidement après administration, pour libérer un des composants androgènes précédemment cités.

4. Utilisation selon la revendication 1, caractérisée en ce que l'androgène d'origine biologique est choisi parmi le 17β-oestradiol, l'oestrone l'oestriol, l'oestrane ou un autre oestrogène d'origine biologique ou est un composé qui se dégrade rapidement après administration, pour libérer un des composants oestrogènes précédemment cités.

5. Utilisation selon la revendication 1, caractérisée en ce que l'oestrogène de synthèse est choisi parmi l'éthinyloestradiol, le mestranol ou un autre oestrogène de synthèse ou est un composé qui se dégrade rapidement après administration, pour libérer un des composants oestrogènes précédemment cités.

6. Utilisation selon au moins une des revendications 1 à 5, caractérisée en ce que le médicament est fabriqué sous forme de pastilles, gélules, dragées, système de thérapie transdermique, ampoules, suppositoires, gels, pommades, gouttes pour le nez, implants, comprimés, ou microsphères biodégradables.
